# EUROPEAN PATENT SPECIFICATION

(11) **EP 1 581 122 B1**
(45) Date of publication and mention of the grant of the patent: **02.11.2006**
(21) Application number: 03767971.9
(22) Date of filing: 09.12.2003
(51) Int. Cl.: A61B 17/04

(54) **A DEVICE FOR TEMPORARILY SHIELDING THE END OF A SURGICAL INSTRUMENT SHARP**
VORRICHTUNG ZUR ZEITWEILIGEN ABSCHIRMUNG DES ENDES EINER NADEL VON EINEM CHIRURGISCHEN INSTRUMENT
DISPOSITIF POUR PROTEGER PROVISOIREMENT L'EXTREMITE D'UNE PARTIE EFFILEE D'UN INSTRUMENT CHIRURGICAL

(30) Priority: 09.12.2002 GB 0228709; 11.08.2003 GB 0318815
(43) Date of publication of application: 05.10.2005
(73) Proprietor: E.D. Medical Ltd, Belfast BT9 6JH (GB)
(72) Inventor: McNICHOLL, Brian, Belfast BT9 6JH (GB)
(74) Representative: Thompson, Trevor George
(86) International application number: PCT/GB2003/005347
(87) International publication number: WO 2004/052214

(56) References cited:
- US-A- 4 949 717
- US-A- 5 908 426
- US-A1- 2002 123 757

## Description

The present invention relates to a device for temporarily shielding the end of a surgical instrument sharp. In particular the present invention relates to a medical device to temporarily shield the end of a suture needle to allow for its removal from tissue safely without allowing the needle to prick the operator or patient unintentionally.

Suturing of body tissue is usually performed with a needle-holder or by a hand-held technique where the needle is guided into the tissue on one side of a wound. Forceps steady the tissue while the needle is inserted with a needle holder. The needle is then pushed beneath the skin to the other side of the wound and then pushed upwards and out of the skin from below. The needle is removed with either forceps or the needle holder which has released the needle after insertion. The suture thread is drawn through the tissue on both sides of the wound by way of being attached to the needle, and a knot is then tied. Tightening the knot then pulls both sides of the wound together.

It is known in the art to provide a surgical instrument sharp end foil for receiving and permanently trapping the free end of a surgical instrument sharp after use. Such a device provides a means for ensuring the permanent disposal and non reuse of surgical instrument sharps.

The document US 5 908 426 discloses a suture needle manipulator for grasping a needle during a surgical procedure.

There is, however, a need for a device which is suitable for housing an end of a surgical instrument sharp, such as a suture needle, to enable the end of the surgical instrument sharp to be temporarily shielded so that it can be used again. Such a device is required, for example, to shield the end of a surgical instrument sharp during a medical procedure.

This is due to the fact that if the end of a surgical instrument sharp is not shielded in any way during a medical procedure, such as suturing, the end of the surgical instrument sharp may stab the operator or their assistant causing an injury. This type of injury can cause damage to the patient or transmission of infection when there is infected blood or body fluids on the needle. Infections such as Hepatitis B and C and Human Immunodeficiency Virus infections are among the more serious of these.

Such injuries usually occur when the operator or their assistant stabs themselves with the end of the surgical instrument sharp while in the process of carrying out medical procedures involving surgical instrument sharps such as in the suturing of a wound. They also occur when a surgical instrument sharp is being brought from one part of the operating theatre to another and being passed from person to person. In a crowded theatre especially when busy, the exposed end of the surgical instrument sharp is a hazard to staff.

The present invention aims to address at least some of the problems associated with the prior art.

Accordingly, the present invention provides a device for temporarily shielding the end of a surgical instrument sharp comprising:
a housing having a proximal end and a distal end;
an opening formed in the distal end of the housing for receiving an end of a surgical instrument sharp therethrough;
a body located in the housing and movable between a first position in which an end of the body obstructs the opening and a second position in which the body is retracted from the opening;
resilient means for biassing the body into the first position; and
actuation means to move the body against the action of the resilient means into the second position.

Preferably the body is a piston with a tapered distal end.

Preferably the distal end of the body does not extend beyond the distal end of the housing when it is in said first position.

The resilient means may be a spring means which is mounted between the proximal end of the body and a stop means within the housing.

Preferably the spring means is a coil spring.

In another embodiment the device may be in combination with forceps and the resilient means can be provided by the resilience of the forceps. In this embodiment the actuation means is preferably a coupling between the body of the device and an arm of the forceps which causes movement of the body when the forceps are operated.

In one embodiment the opening is defined by a re-entrant end wall portion of the housing.

In a further embodiment the inner surface of the housing tapers inwardly towards the opening.

In another embodiment the actuation means comprises a pullwire secured to the body. Advantageously, a pullwire may be employed in embodiments whereby the housing curves through angles up to 90°, and larger angles if pulleys are provided to mount the pullwire.

In another embodiment the body can wobble from side to side.

Viewed from a further aspect, the present invention relates to a device for temporarily shielding the end of a surgical instrument sharp comprising: a housing having a proximal end and a distal end; an opening formed in the distal end of the housing for receiving an end of a surgical instrument sharp therethrough; a body located in the housing and movable between a first position in which an end of the body obstructs the opening and a second position in which the body is retracted from the opening; first and second arms movable relative to each other; a support bar to couple the body to one of said first and second arms; wherein movement of the first and second arms relative to each other causes the support bar to move the body between said first and second positions. The support bar and the body may be formed integrally as a single component.

For a better understanding of the invention it will now be described, by way of example, with reference to the accompanying drawings, in which:
- Fig. 1: is a partial cross section of one embodiment of the device;
- Fig. 2: is a partial cross section of the device showing the free end of a suture needle lodged in the device.
- Fig. 3: is a perspective view of the device in use showing the device preparing to receive a suture needle from a wound.
- Fig. 4: is a schematic cross section of a further embodiment of the device when the device is angled.
- Fig. 5: is a perspective view of a further embodiment of the device mounted on forceps in a first position.
- Fig. 6: is a perspective view of the embodiment of the device shown in Fig. 5 in a second position.
- Fig. 7: is a cross-sectional view of a still further embodiment of the device mounted on forceps in a first position.
- Fig. 8: is a perspective view of the embodiment of the device shown in Figure 7 in a second position.
- Fig.9: is a perspective view of a further embodiment of the device mounted on forceps.

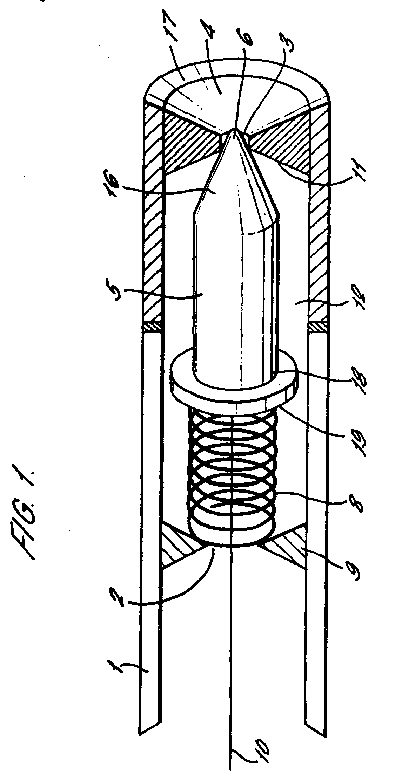

As shown in Figures 1 to 6 the device comprises a housing 1 in the form of a tubular wall which is shown circular in cross section but could be any other desired shape. The housing 1 is typically made of metal.

The housing 1 can be formed of one complete part or it can be formed of separate parts, for example two separate parts as shown in Figures 1 to 3. If the housing 1 is formed of separate parts then any suitable means can be used to join the separate parts rigidly together. For example, if the separate parts are metal, they can be welded together.

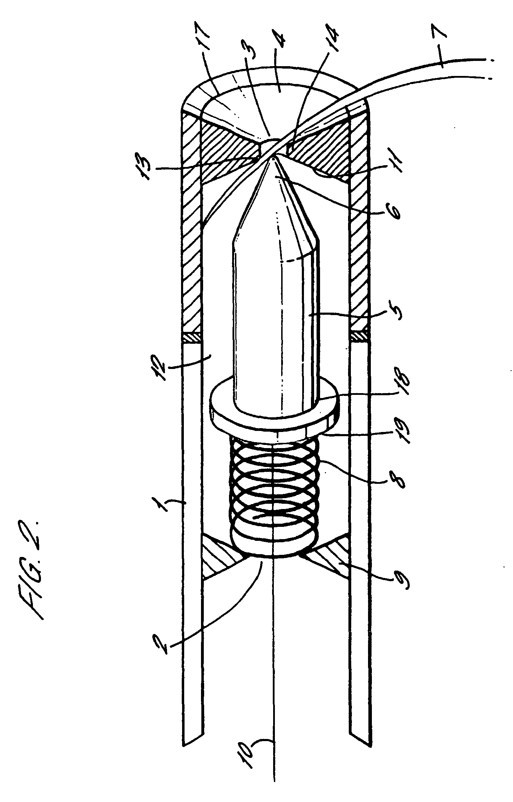

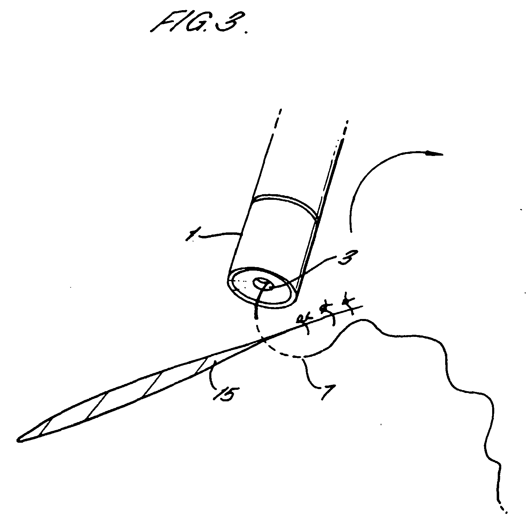

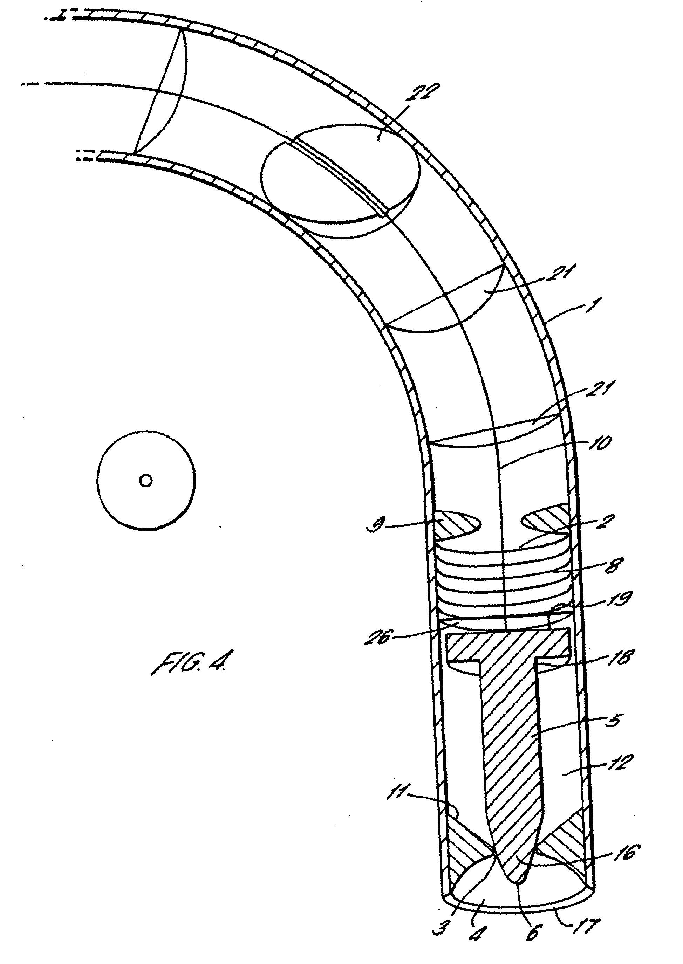

The device can be completely straight, as shown in Figures 1 to 3, 5 and 6 or angled, as shown in Figure 4, depending on the demands of different wounds. An angled device enables the operator to remove needles from tissues of different depths or positions where access to the tissue being sutured is restricted.

It is possible that where the housing 1 is formed of two separate parts, these parts can project at a variable angle from each other.

The housing 1 has a proximal end and a distal end. The distal end of the device is the end shown in Figures 1 and 2 which is towards the right hand side of the page and the proximal end of the device is towards the left hand side of the page.

The housing 1 is partially closed at the distal end by a wall 11 with a central opening 3. The opening 3 is defined by a re-entrant end wall portion 4 which shapes back into the housing 1 so that the opening 3 is recessed from the distal extremity of the housing 1. The size of the opening 3 is dependent on the surgical instrument sharp which it is intended to receive.

The inner surface of the housing 1 tapers inwardly towards opening 3.

As shown in Figures 1, 2 and 4 the housing 1 defines a chamber 12 which contains a body in the form of a piston 5. The piston 5 has a proximal end 18 preferably having a larger cross-section than the rest of the piston 5. The proximal end 18 of the piston 5 is dimensioned to ensure a relatively tight fit within the housing 1, whilst still being movable axially within the chamber 12. The proximal end 18 of the piston 5 is therefore usually of the same cross-sectional shape as the chamber 12, although this would not be essential. Figures 1 and 2 over emphasize the gap between the proximal end 18 of the piston 5 and the sides of the housing 1. In reality there would be a much smaller gap, to ensure a tight fit thus allowing the proximal end 18 of the piston 5 to move within the chamber 12, keeping to the midline.

In a preferred embodiment a washer 26, as shown in Figure 4, may be situated between the proximal end 18 of the piston 5 and the distal end 19 of the spring 8. This ensures that the piston 5 when retracted does not slip inside the spring 8.

The rest of the body of the piston 5 is preferably dimensioned to ensure that there is not a tight fit between the sides of the housing 1 and the piston 5 but instead that there is adequate space between the body of the piston 5 and the sides of the housing 1 to ensure that a needle can be lodged between the sides of the piston 5 and the sides of the housing 1. Preferably, the diameter of the piston should be between 50 to 70 % of the internal diameter of chamber 12.

The piston 5 can take up any length within the housing 1 provided that some axial movement is still possible.

The piston 5 has a tapered distal end 16. The tip 6 of the tapered distal end 16 of the piston 5 is of such a shape and size that when the piston 5 is at rest, the tip 6 lodges in the opening 3, with a small portion of the tip 6 protruding through the opening 3.

The tip 6 of the piston 5 whilst pointed is shaped such that it is not sharp enough to cause any harm.

Attached to the proximal end 18 of the piston 5 is a resilient member such as a coil spring 8 which is partly in compression. The proximal end 2 of the spring 8 is lodged against a stop 9 on the inner wall of the housing 1. The distal end 19 of the spring 8 is attached to the proximal end 18 of the piston 5. The spring 8 is dimensioned to ensure a tight fit within the housing 1, whilst still being movable axially within the chamber 12. The spring 8 is therefore usually of the same cross-sectional shape as the chamber 12 although this would not be essential. Figures 1 and 2 over emphasize the gap between the spring 8 and the sides of the housing 1. In reality there would be a much smaller gap, to ensure a tight fit.

Actuation means such as a lever or a pullwire 10 is secured to the proximal end 18 of the piston 5. Actuation means such as handle, not shown in any of the Figures, could instead be attached directly to the spring 8.

Depending on the depth of the wound, the distance of the housing 1 from the surgeon's hand and/or the means for activating the actuation means can be varied by lengthening the housing 1, as shown in Figure 4. For example, for keyhole surgery this may be 30 to 60 cm (1 to 2 feet) in length.

As shown in Figure 4, the housing 1 may be curved, i.e. the device may be angled. In these angled arrangements, pulley 21 and/or tracks 22 can be spaced inside the housing 1 to reduce the likelihood of the pullwire actuation means snagging or catching in the housing 1. Preferably the pulleys 21 and/or tracks 22 are situated in the housing 1 after the proximal end 2 of the spring 8. The pulleys 21 can be in the form of circular pieces of material with a central hole, preferably made of metal or plastic, by which the pullwire 10 can pass through. The pulleys 21 have the same cross section as the chamber 12 formed by the housing 1 to ensure that they are lodged against the sides of the housing 1. The tracks 22 may be beads having a channel defined therein through which the pullwire 10 runs to enable the pullwire 10 to travel freely round corners. At least one pulley 21 and/or at least one track 22 is/are preferably provided when the device is angled. If a plurality of pulleys and/or tracks are used, they are preferably evenly spaced in the housing.

The general use of the device will now be explained. The use of the device with specific shaped needles and when attached to forceps is explained in more detail later.

In use the user will pull the piston 5 back against the force of the spring 8 using the pullwire 10.

The user can pull the pullwire 10 directly with his hand, or via an actuator or any other suitable mechanism which can be used to pull the pullwire 10. For example, the user may pull a bead or a ring attached to the pullwire 10 or a trigger-like mechanism. None of these mechanisms are shown in the Figures.

This action compresses the spring 8 to a greater extent and causes the piston 5 to retract within the housing 1 to allow the tip 6 of the piston 5 to disengage from the opening 3.

The opening 3 is then clear of obstruction to allow the end of a surgical instrument sharp such as a needle 7 to enter.

The re-entrant end wall portion 4 ensures that should the end of the needle 7 not enter the opening 3 immediately, but touch the outside edge 17 of the housing 1 instead, the sloping surface will deflect the end of the needle 7, and channel it into the housing 1 via the opening 3.

The loose fitting of the body of the piston 5 enables the body of the piston 5 to move from side to side i.e. to wobble. The sideways movement or "wobble" of the piston 5 reduces the likelihood of the needle 7 lodging against the piston 5 and instead enables the needle 7 to slide off and enter further alongside the piston 5 into the chamber 12.

The pointed tip 6 of the piston 5 ensures also that the sharp tip of the end of the needle 7 does not lodge against it, but deflects the needle tip sideways into the housing 1, allowing the needle 7 to enter further inside the housing 1, as seen in Figure 2.

Once the free end of the needle 7 has entered the opening 3 and is within the housing 1 the user will let go of the pullwire 10 which allows the spring 8 to advance the piston 5. The inner walls of the housing 1 which taper towards opening 3 guide the tapered distal end 16 and therefore the tip 6 of the piston 5 towards the opening 3 as it advances. This clamps the body of the needle 7 against the inner edges of the opening 3.

The piston 5 with its ability for sideways movement will accommodate itself to the lie of the needle 7 to a certain extent when it descends to ensure that the needle 7 does not move excessively on clamping. If the piston 5 was a tight fit along its length in the chamber 12 it could force the needle to move excessively on clamping, the force along the shank of the needle causing the proximal end of the needle to move within the tissue. In some tissues, for example, blood vessels, this may cause the vessel to tear.

The end of the needle 7 is now clamped within the housing 1, as shown in Figure 2, so that the end of the needle 7 is covered and cannot cause injury.

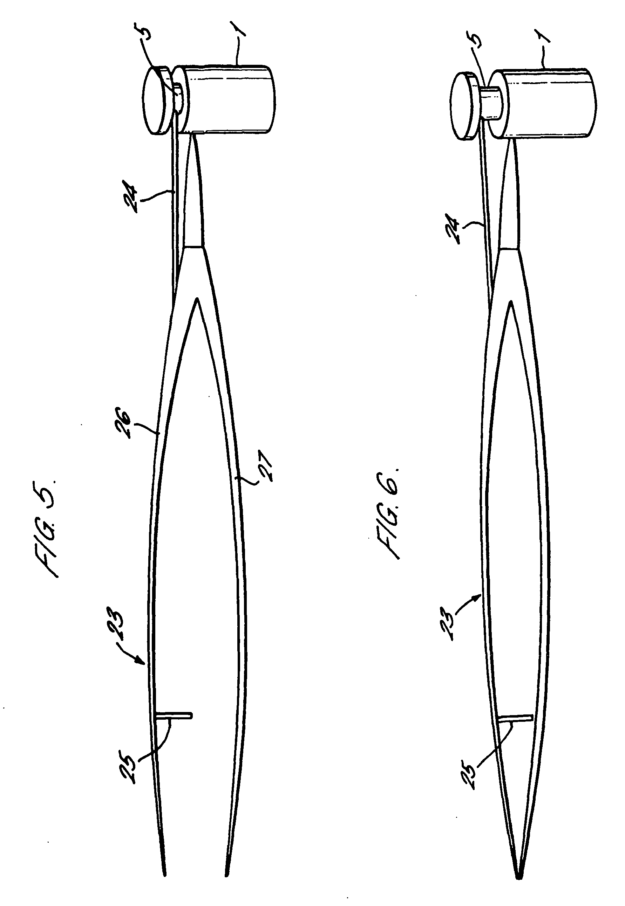

As explained earlier, suturing of body tissue is usually performed with a needle holder or by a hand held technique where the needle is guided into the tissue on one side of a wound. Forceps 23, as shown in Figure 5, having first and second spring-loaded arms 26,27 may be used to steady the tissue while the needle 7 is inserted with a needle holder. The needle 7 is then pushed beneath the skin to the other side of the wound and then pushed upwards and out of the skin from below.

An alternative embodiment of the invention is shown in Figure 5. In this embodiment the housing 1 of the device is fixed to the body of the forceps 23 at the junction between the first and second arms 26, 27. A support bar 24, mounted on the first arm 26 of the forceps 23, is attached to the piston 5 of the device using any suitable means. The attachment of the device to the forceps can be permanent, such as by welding or by brazing, or the attachment can be temporary, i.e. the device can be detachable.

In use the resilient action is provided by the resting tension of the forceps 23. The first and second arms 26, 27 of the forceps 23 are springingly biassed open in the resting position as shown in Figure 5. In the open position, tension is transmitted via the support bar 24 to bias the piston 5 towards the distal end of the housing 1 to obstruct the opening 3.

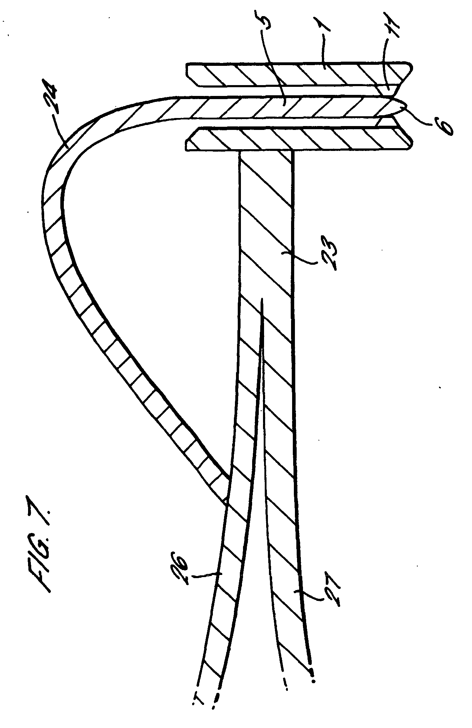

The piston 5 is moved upwards away from the opening 3 in the housing 1 by compression of the first and second arms 26, 27 of the forceps 23 by the user. This compression causes the angle of the support bar 24 to change thereby causing the piston 5 to lift up, as shown in Figure 6.

The tension required to cause the piston 5 to lift up can be adjusted by varying axially the position of the piston 5 relative to the support bar 24. This adjustment can be achieved by providing screw threads on the proximal end 18 of the piston 5 where it is mounted in a collar provided on the support bar 24. By varying the position of the piston 5 relative to the collar the resting tension of the piston 5 and also of a set of forceps teeth 25 provided on the first arm 26 can be adjusted. The piston 5 can still maintain its wobble at the distal end 6 thereof as the threads holding the piston are not tightened, i.e. they are loose and allow a small amount of lateral movement when the piston is lifted up and hanging free.

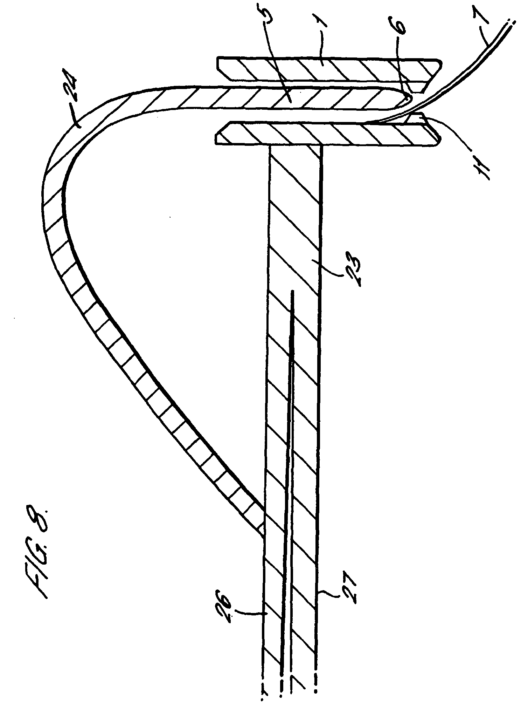

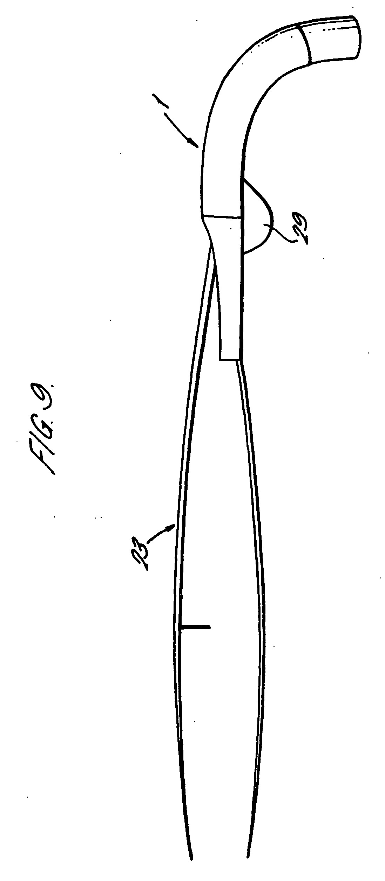

An alternative embodiment of the device mounted on forceps 23 is shown in Figures 7 and 8. The forceps 23 comprise first and second resilient arms 26,27 which are biassed towards an open position, as shown in Figure 7. In this embodiment, the piston 5 and support bar 24 are formed integrally and the end of the support bar 24 distal from the piston 5 is attached to the first arm 26 of the forceps 23. It will be appreciated that the forceps 23 or first arm 26 may be formed integrally with the support bar 24 and piston 5.

As shown in Figure 7, when the first and second arms 26,27 are in their open position, the piston 5 is located in an advanced position in the housing 1 such that the opening 3 defined in an end therefore is at least partially obstructed. Preferably, the end 6 of the piston 5 abuts the wall 11 of the housing 1 when the forceps 23 are in an open position.

When the first and second arms 26,27 of the forceps 23 are displaced towards each other, the support bar 24 is displaced and the piston 5 moves to a retracted position away from the opening 3, as shown in Figure 8. With the piston 5 in this retracted position, a needle 7 or other sharp may be received into the housing 1 through the opening 3. The first and second arms 26,27 may then be released and allowed to return to their open position. This movement of the first and second arms 26,27 towards their open position causes the piston 5 to be biassed towards the opening 3 thereby trapping the needle 7 in the housing 1.

It will be appreciated that in this embodiment the body 5 does not move along, or parallel to, the longitudinal axis of the housing 1. Rather, the body 5 follows an arcuate path. This movement allows the needle 7 more readily to be received into the housing 1 to the side of the body 5 closest to the forceps 23, as shown in Figure 8. This is particularly desirable as the needle 7 is likely to be oriented upwardly when, for example, a wound is being closed and the device may be used without obstructing the user's view. In the offset open position, as shown in Figure 8, the portion of the wall 11 closest to the tip 6 of the body 5 advantageously shields the body 5 and, therefore, reduces the likelihood of the needle 7 being obstructed as it enters the housing 1.

In this arrangement, the resilience of the support bar 24 limits transverse movement of the piston 5, relative to the housing 1 and allows the needle 7 to be securely retained in the housing 1.

The forceps 23, support arm 24, piston 5 and housing 1 may be formed from a plastics material or metal. The components may be formed integrally as a single unit or formed separately and then assembled.

It will be appreciated that the device shown in Figures 7 and 8 need not be provided on a pair of forceps 23. Rather, the device may be formed as a dedicated instrument having first and second arms 26,27 which do not form part of a pair of forceps 3. It will, of course, be appreciated that the embodiment shown in Figures 5 and 6 also need not be provided on a pair of forceps 23.

A further embodiment of the device, intended to be used only once prior to disposal along with the medical sharp retained therein, is shown in Fig.9. The disposable device is detachably mounted on the end of the forceps 23 and has a housing 1 which is curved through 90° for use in stitching uncomplicated or readily accessible wounds. The housing 1 is preferably made of a plastic material to reduce the cost of the device. A metal end-piece (not shown) is provided in the housing 1 to define the wall 11 having the opening 3 therein. The piston is preferably also metal for engaging positively a surgical instrument sharp inserted into the device. A slidable actuating member 29 is provided on the housing 1 to retract the piston into the housing.

Disposable devices for use either with forceps or independently of forceps, having housings 1 with or without curved profiles, are envisaged.

In standard suturing a curved needle is used as shown in Figures 2 and 3. A curved needle 7 will come out of a wound along a curved path. To facilitate this, the user of the device will carry out a pill-rolling action with his fingers and a supinating action of the wrist causing the forceps and the device of the present invention to facilitate this curved path. This rotating action will also exert a flexing force on the needle 7 helping it to lodge in the housing 1 as shown in Figure 2.

Firstly the shank of the needle 7 is clamped at the inner margin 13 of the opening 3 by the piston 5. Secondly the tip of the end of the needle may be lodged against the inner side surface of the housing 1. Third, the shank of the needle 7 may lodge against the outer margin 14 of the opening 3. Fourth, the end of the shank of the needle 7 is held within the tissue of the wound 15. This rotating hand action, along a path similar to the concave curve of the needle 7, will tend to flex the needle 7, increasing its concavity. The tissues will require torque by the device to extract the needle 7. As the tissue friction varies, depending on the type of wound, so will the torque required. The greater the torque required the more securely the needle 7 is lodged against the surfaces it rest upon. This twisting force will help to lodge the needle 7 firmly in the device and reduce the likelihood of it slipping out where there is significant resistance from the tissue.

When used with straight needles the tip of the end of the needle 7 again lodges against the side of the inner surface of the housing 1 but because there is no curvature the shank does not. The straight needle is held by the piston 5, and when a torque is created, the tip of the end of the needle 7 lodges against the inner surfaces of the housing 1 and against the margins 14 of the opening 3.

It is then possible, for example, when used in suturing for the needle 7 to be pulled by the user of the device from the tissue along with the thread, and a knot tied.

When the needle 7 is ready to be discarded it can be carried in the device to the nearest disposal unit. It can be passed safely to another member of the surgical team for disposal.

The housing 1 of the device can be configured so that it may be removably attached to other medical instruments, for example the end of a forceps or it may be configured for stand alone use such as in keyhole surgery.

Even though in a preferred embodiment the invention is described with reference to a needle used for the application of sutures to wounds of the skin or deeper tissues in a wide variety of surgical procedures it is to be understood that the present invention can also be applied to any surgical instrument sharp, for example hypodermic needles, syringes, trocars and stylets.

It will be appreciated that the device described herein may be used for surgical needles, hollow needles suitable for taking blood, wires and rods. The device is also suitable for trapping the end of a thread or a string thereby further increasing the usefulness of the device for wound closure. An enlarged version of the device is also envisaged to assist in rod handling.

The device may also be used to shield a sewing needle when cloth or fabric is being stitched. The device may also be used to shield wires and the like during, for example, the assembly of electrical circuit boards.

## Claims

1. A device for temporarily shielding the end of a surgical instrument sharp comprising:
a housing (1) having a proximal end and a distal end;
an opening (3) formed in the distal end of the housing for receiving an end of a surgical instrument sharp (7) therethrough;
a body (5) located in the housing and movable between a first position in which an end of the body obstructs the opening and a second position in which the body is retracted from the opening;
resilient means (8) for biasing the body into the first position; and
actuation means (10; 24) to move the body against the action of the resilient means (10) into the second position.

2. A device as claims in claim 1 wherein the body is a piston (5) with a tapered distal end (16).

3. A device as claimed in any one of the preceding claims wherein the resilient means (8) is a spring means which is mounted between the proximal end of the body (5) and a stop means (9) within the housing.

4. A device as claimed in claim 3 wherein the spring means (8) is a coil spring.

5. A device as claimed in claim 1 or 2 in combination with forceps (23), wherein the device is mounted on the forceps and the resilient means (8) is provided by the resilience of the forceps.

6. A device as claimed in claim 5 wherein said actuation means is a coupling (10; 24) provided between said body of the device and an arm (26) of the forceps.

7. A device as claimed in claim 6, wherein the coupling (10; 24) is formed integrally with the body (5).

8. A device as claimed in any one of the preceding claims wherein the opening (3) is defined by a reentrant end wall (11) portion of the housing.

9. A device as claimed in any one of the preceding claims wherein the inner surface of the housing (1) tapers inwardly towards the opening (3).

10. A device as claimed in any one of the preceding claims wherein the actuation means comprises a pullwire (10) secured to the body (5).

11. A device as claimed in any one of the preceding claims wherein at least the end of the body (5) which obstructs the opening (3) can wobble from side to side when the body is in said second position.

12. A device as claimed in claim 1 further comprising first and second arms (26, 27) and said actuation means (10; 24) is coupled to at least one of said first and second arms.

13. A device as claimed in claim 12 wherein said actuation means is a coupling (10; 24) provided between said body (1) of the device and the at least one of said first and second arms (26, 27).

14. A device as claimed in claim 13 wherein said coupling is a support bar (24).

15. A device as claimed in claim 12, 13 or 14 wherein said actuation means (10; 24) is integrally formed with said body (5).

16. A device as claimed in any one of claims 12 to 15 wherein said first and second arms (26, 27) are spring biased towards or away from each other and the resilient means (8) is provided by the spring bias of said first and second arms.

17. A device for temporarily shielding the end of a surgical instrument sharp (7) comprising:
a housing (1) having a proximal end and a distal end;
an opening (3) formed in the distal end of the housing for receiving an end of a surgical instrument sharp therethrough;
a body (5) located in the housing and movable between a first position in which an end of the body obstructs the opening and a second position in which the body is retracted from the opening;
first and second arms (26, 27) movable relative to each other;
a support bar (24) to couple the body to one of said first and second arms;
wherein movement of the first and second arms relative to each other causes the support bar to move the body between said first and second positions.

## Patentansprüche

1. Einrichtung zum temporären Abschirmen des Endes einer chirurgischen Instrumentenspitze/schneide, um fassend:
ein Gehäuse (1) mit einem proximalen Ende und einem distalen Ende;
eine Öffnung (3), die in dem distalen Ende des Gehäuses ausgebildet ist, um ein Ende einer chirurgischen Instrumentenspitze/schneide (7) durch sie hindurch aufzunehmen;
einen Körper (5), der in dem Gehäuse angeordnet und zwischen einer ersten Position, in der ein Ende des Körpers die Öffnung versperrt, und einer zweiten Position, in der der Körper von der Öffnung zurückgezogen ist, bewegbar ist;
elastische Mittel (8), um den Körper in der ersten Position vorzuspannen; und
Betätigungsmittel (10; 24), um den Körper gegen die Wirkung der elastischen Mittel (10) in die zweite Position zu bewegen.

2. Einrichtung, wie in Anspruch 1 beansprucht, wobei der Körper ein Kolben (5) mit einem sich verjüngenden distalen Ende (16) ist.

3. Einrichtung, wie in einem der vorstehenden Ansprüche beansprucht, wobei das elastische Mittel (8) ein Federmittel ist, das zwischen dem proximalen Ende des Körpers (5) und einem Stoppmittel (9) in dem Gehäuse angebracht ist.

4. Einrichtung, wie in Anspruch 3 beansprucht, wobei das Federmittel (8) eine Spiralfeder ist.

5. Einrichtung, wie in Anspruch 1 oder 2 beansprucht, in Kombination mit einem Forceps (23), wobei die Einrichtung an dem Forceps angebracht ist und das elastische Mittel (8) durch die Elastizität des Forceps bereitgestellt wird.

6. Einrichtung, wie in Anspruch 5 beansprucht, wobei das Betätigungsmittel eine Kupplung (10; 24), ist, die zwischen dem Körper der Einrichtung und einem Arm (26) des Forceps vorgesehen ist.

7. Einrichtung, wie in Anspruch 6 beansprucht, wobei die Kupplung (10; 24) integral mit dem Körper (5) ausgebildet ist.

8. Einrichtung, wie in einem der vorstehenden Ansprüche beansprucht, wobei die Öffnung (3) durch einen ablaufinvarianten Endwandungs-(11)-Abschnitt des Gehäuses definiert ist.

9. Einrichtung, wie in einem der vorstehenden Ansprüche beansprucht, wobei sich die innere Oberfläche des Gehäuses (1) in Richtung der Öffnung (3) verjüngt.

10. Einrichtung, wie in einem der vorstehenden Ansprüche beansprucht, wobei das Betätigungsmittel einen Zugdraht (10) umfasst, der an dem Körper (5) befestigt ist.

11. Einrichtung, wie in einem der vorstehenden Ansprüche beansprucht, wobei sich zumindest das Ende des Körpers (5), das die Öffnung (3) versperrt, von Seite zu Seite hin- und herbewegen kann, wenn sich der Körper in der zweiten Position befindet.

12. Einrichtung, wie in Anspruch 1 beansprucht, die des weiteren erste und zweite Arme (26, 27) umfasst und bei der das Betätigungsmittel (10; 24) mit dem ersten und/oder dem zweiten Arm verbunden ist.

13. Einrichtung, wie in Anspruch 12 beansprucht, wobei das Betätigungsmittel eine Kupplung (10; 24) ist, die zwischen dem Körper (1) der Einrichtung und dem ersten und/oder zweiten Arm (26, 27) vorgesehen ist.

14. Einrichtung, wie in Anspruch 13 beansprucht, wobei die Kupplung eine Stützstange (24) ist.

15. Einrichtung, wie in Anspruch 12, 13 oder 14 beansprucht, wobei das Betätigungsmittel (10; 24) integral mit dem Körper (5) ausgebildet ist.

16. Einrichtung, wie in einem der Ansprüche 12 bis 15 beansprucht, wobei der erste und der zweite Arm (26, 27) aufeinander zu oder voneinander weg gespannt sind und das elastische Mittel (8) von der Federspannung des ersten und zweiten Arms zur Verfügung gestellt wird.

17. Einrichtung zum temporären Abschirmen des Endes einer chirurgischen Instrumentenspitze/schneide, umfassend:
ein Gehäuse (1) mit einem proximalen Ende und einem distalen Ende;
eine Öffnung (3), die in dem distalen Ende des Gehäuses ausgebildet ist, um ein Ende einer chirurgischen Instrumentenspitze/schneide (7) durch sie hindurch aufzunehmen;
einen Körper (5), der in dem Gehäuse angeordnet und zwischen einer ersten Position, in der ein Ende des Körpers die Öffnung versperrt, und einer zweiten Position, in der der Körper von der Öffnung zurückgezogen ist, bewegbar ist;
einen ersten und einen zweiten Arm (26, 27), die relativ zueinander bewegbar sind;
eine Stützstange (24), um den Körper mit dem ersten oder dem zweiten Arm zu verbinden;
wobei eine Bewegung des ersten und des zweiten Arms relativ zueinander bewirkt, dass die Stützstange den Körper zwischen der ersten und der zweiten Position bewegt.

## Revendications

1. Dispositif pour protéger temporairement l'extrémité d'une partie effilée d'un instrument chirurgical, comprenant :
un boîtier (1) ayant une extrémité proximale et une extrémité distale;
une ouverture (3) formée dans l'extrémité distale du boîtier pour y recevoir, à travers, une extrémité d'une partie effilée (7) d'un instrument chirurgical;
un corps (5) placé dans le boîtier et pouvant être déplacé entre une première position, dans laquelle une extrémité du corps bouche l'ouverture, et une deuxième position, dans laquelle le corps est retiré de l'ouverture;
un moyen élastique (8) pour presser le corps dans la première position; et
un moyen d'actionnement (10; 24) pour déplacer le corps à l'encontre de l'action du moyen élastique (10) dans la deuxième position.

2. Dispositif selon la revendication 1, dans lequel le corps est un piston (5) avec une extrémité distale effilée (16).

3. Dispositif selon l'une quelconque des revendications précédentes, dans lequel le moyen élastique (8) est un moyen à ressort qui est monté entre l'extrémité proximale du corps (5) et un moyen d'arrêt (9) dans le boîtier.

4. Dispositif selon la revendication 3, dans lequel le moyen à ressort (8) est un ressort hélicoïdal.

5. Dispositif selon la revendication 1 ou 2, en combinaison avec un forceps (23), dans lequel le dispositif est monté sur le forceps et le moyen élastique (8) est fourni par l'élasticité du forceps.

6. Dispositif selon la revendication 5, dans lequel ledit moyen d'actionnement est un couplage (10; 24) aménagé entre ledit corps du dispositif et un bras (26) du forceps.

7. Dispositif selon la revendication 6, dans lequel le couplage (10; 24) est formé d'une seule pièce avec le corps (5).

8. Dispositif selon l'une quelconque des revendications précédentes, dans lequel l'ouverture (3) est définie par une partie de paroi d'extrémité rentrante (11) du boîtier.

9. Dispositif selon l'une quelconque des revendications précédentes, dans lequel la surface interne du boîtier (1) s'effile vers l'intérieur en direction de l'ouverture (3).

10. Dispositif selon l'une quelconque des revendications précédentes, dans lequel le moyen d'actionnement comprend un fil de traction (10) fixé au corps (5).

11. Dispositif selon l'une quelconque des revendications précédentes, dans lequel au moins l'extrémité du corps (5), qui bouche l'ouverture (3), peut vaciller d'un côté à l'autre lorsque le corps est dans ladite deuxième position.

12. Dispositif selon la revendication 1, comprenant en outre un premier et un deuxième bras (26, 27) et ledit moyen d'actionnement (10; 24) est couplé à au moins l'un desdits premier et deuxième bras.

13. Dispositif selon la revendication 12, dans lequel ledit moyen d'actionnement est un couplage (10; 24) aménagé entre ledit corps (1) du dispositif et le au moins un desdits premier et deuxième bras (26, 27).

14. Dispositif selon la revendication 13, dans lequel ledit couplage est une barre de support (24).

15. Dispositif selon la revendication 12, 13 ou 14, dans lequel ledit moyen d'actionnement (10; 24) est formé d'une seule pièce avec ledit corps (5).

16. Dispositif selon l'une quelconque des revendications précédentes 12 à 15, dans lequel lesdits premier et deuxième bras (26, 27) sont pressés par un ressort l'un vers l'autre ou l'un à l'opposé de l'autre et le moyen élastique (8) est fourni par la sollicitation du ressort desdits premier et deuxième bras.

17. Dispositif pour protéger temporairement l'extrémité d'une partie effilée (7) d'un instrument chirurgical, comprenant :
un boîtier (1) ayant une extrémité proximale et une extrémité distale;
une ouverture (3) formée dans l'extrémité distale du boîtier pour y recevoir, à travers, une extrémité d'une partie effilée (7) d'un instrument chirurgical;
un corps (5) placé dans le boîtier et pouvant être déplacé d'une première position, dans laquelle une extrémité du corps bouche l'ouverture, et une deuxième position, dans laquelle le corps est retiré de l'ouverture;
un premier et un deuxième bras (26, 27) pouvant être déplacés l'un par rapport à l'autre;
une barre de support (24) pour coupler le corps à l'un desdits premier et deuxième bras;
dans lequel le mouvement des premier et deuxième bras l'un par rapport à l'autre entraîne le déplacement par la barre de support du corps entre lesdites première et deuxième positions.
